# FASCICULE DE BREVET EUROPEEN

(11) **EP 4 408 497 B1**
(45) Date de publication et mention de la délivrance du brevet: **25.06.2025**
(21) Numéro de dépôt: 22797429.2
(22) Date de dépôt: 03.10.2022
(51) Int. Cl.: A61M 1/00, A61B 90/00, B09B 3/80, B26D 1/15, B09B 3/35, B26D 7/06, B26D 7/18, B65F 1/10, A61L 11/00

(54) **SYSTÈME DE TRAITEMENT DE POCHES D'ASPIRATION**
SYSTEM ZUR VERARBEITUNG VON SAUGBEUTELN
SYSTEM FOR PROCESSING SUCTION BAGS

(30) Priorité: 01.10.2021 FR 2110410
(43) Date de publication de la demande: 07.08.2024
(73) Titulaire: Def'Hygie, 69480 Amberieux d'Azergues (FR)
(72) Inventeur: DUMONCEAU, Benoit, 69480 Lachassagne (FR); POYARD, Raphaël, 69380 Chessy (FR); MAIROT, Laurent, 34000 Montpellier (FR)
(74) Mandataire: Germain Maureau
(86) Numéro de dépôt international: PCT/FR2022/051867
(87) Numéro de publication internationale: WO 2023/052737

(56) Documents cités:
- CN-U- 212 419 000
- US-A1- 2006 060 619
- US-B1- 8 197 174

## Description

### Domaine technique

L'invention concerne un système de traitement, destiné à ouvrir et à traiter des poches d'aspiration pouvant contenir des liquides et/ou des fluides corporels.

L'invention trouve une application favorite, et non limitative, dans le traitement de poches d'aspiration pouvant être utilisées dans le domaine médical et hospitalier afin de récupérer des fluides corporels provenant des patients. Des applications sont envisageables dans le domaine industriel par exemple afin de séparer et de traiter des poches et leur contenu.

En effet, la séparation automatisée d'un fluide initialement situé dans une enveloppe permet de limiter le contact entre un utilisateur et le fluide contenu dans l'enveloppe, ce qui est avantageux notamment lorsque les fluides peuvent présenter des risques quelconques vis-à-vis de l'utilisateur.

### Etat de la technique

Il est ainsi connu de l'art antérieur et du document CN212419000U d'employer un système de traitement comportant un organe de coupe monté mobile en rotation autour d'un axe vertical de manière à définir un plan de coupe horizontal, l'organe de coupe étant configuré pour venir ouvrir dans la largeur, des poches d'aspiration préalablement positionnées dans un bâti principal.

Ce type de système de traitement permet d'effectuer l'ouverture de poches d'aspiration aisément et sans contact d'un utilisateur avec le contenu des poches d'aspiration. Cependant, un tel système de traitement présente des inconvénients majeurs, le premier étant l'impossibilité d'ajouter une ou plusieurs poches d'aspiration dans le bâti principal tant que le cycle de traitement des poches d'aspiration déjà en place n'est pas terminé. Par ailleurs, l'ouverture de poches d'aspiration dans leur largeur n'est pas optimale, puisqu'une partie du contenu de la poche d'aspiration peut subsister dans la partie inférieure de la poche d'aspiration après une ouverture de cette dernière dans la largeur.

### Résumé de l'invention

Le problème technique à la base de l'invention consiste donc à fournir un système de traitement de poches d'aspiration permettant d'assurer une vidange améliorée des poches d'aspiration et autorisant l'insertion de poches d'aspiration à l'intérieur du système de traitement simultanément au traitement de poches déjà présentes dans le système de traitement, tout en évitant un contact d'un utilisateur avec le contenu des poches d'aspiration.

A cet effet, la présente invention concerne un système de traitement de poches d'aspiration, et plus particulièrement de poches d'aspiration contenant des déchets liquides, et par exemple des déchets médicaux liquides, comprenant :
- un bâti comportant une zone de chargement configurée pour permettre un chargement de poches d'aspiration au sein du bâti, une zone de découpe dans laquelle sont destinées à être découpées des poches d'aspiration, une zone de traitement dans laquelle des déchets liquides, provenant de poches d'aspiration découpées, sont destinés à être traités, et une zone de stockage dans laquelle sont destinées à être stockées des poches d'aspiration traitées,
- un support rotatif qui est logé au moins en partie dans le bâti et qui est monté mobile en rotation autour d'un premier axe de rotation, le support rotatif comprenant plusieurs emplacements de réception configurés chacun pour accueillir une poche d'aspiration, le support rotatif étant configuré pour déplacer des poches d'aspiration reçues dans les emplacements de réception depuis la zone de chargement vers la zone de découpe puis vers la zone de stockage,
- un organe de coupe qui est situé dans la zone de découpe et qui est monté mobile en rotation autour d'un deuxième axe de rotation qui est sensiblement horizontal, l'organe de coupe définissant un plan de coupe qui est sensiblement vertical et étant configuré pour découper au moins une poche d'aspiration reçue dans un emplacement de réception et située dans la zone de découpe.

Ainsi, le système de traitement selon l'invention permet d'effectuer l'ouverture de poches d'aspiration aisément et sans contact d'un utilisateur avec le contenu des poches d'aspiration, tout en permettant d'ajouter une ou plusieurs poches d'aspiration dans le bâti simultanément au traitement d'au moins une poche d'aspiration située dans la zone de découpe et/ou à l'évacuation d'une poche d'aspiration dans la zone de stockage. Par ailleurs, l'ouverture de poches d'aspiration dans leur longueur permet de récupérer de façon optimale le contenu d'une telle poche d'aspiration.

Dans le présent texte, on entend par « déchets médicaux liquides » notamment des déchets liquides à risque infectieux (DASRI) et/ou à risque chimique provenant par exemple de différents établissements de santé et médico-sociaux, d'industrie, ainsi que des fluides corporels.

Le système de traitement peut, en outre, présenter une ou plusieurs des caractéristiques suivantes, qui peuvent être prises seules ou en combinaison.

Selon un mode de réalisation, le premier axe de rotation est sensiblement vertical.

Selon un mode de réalisation, le plan de coupe est tangent avec un cercle virtuel dont le centre est situé sur le premier axe de rotation du support rotatif. En d'autres termes, le deuxième axe de rotation de l'organe de coupe s'étend radialement par rapport au premier axe de rotation du support rotatif.

Selon un mode de réalisation, l'organe de coupe est monté mobile par rapport au bâti selon une direction de déplacement qui est sensiblement verticale. Ainsi, l'organe de coupe permet de découper de manière optimale des poches d'aspiration de tailles différentes.

Selon un mode de réalisation, la zone de chargement du système de traitement comporte au moins une ouverture d'insertion supérieure configurée pour permettre de positionner simultanément une pluralité de poches d'aspiration dans des emplacements de réception du support rotatif situés en regard de l'au moins une ouverture d'insertion supérieure.

Selon un mode de réalisation, les emplacements de réception sont angulairement décalés les uns par rapport aux autres vis-à-vis du premier axe de rotation.

Selon un mode de réalisation, les emplacements de réception sont situés à une même distance du premier axe de rotation.

Selon un mode de réalisation, le système de traitement comporte entre deux et dix emplacements de réceptions, et par exemple six emplacements de réception.

Selon un mode de réalisation, le système de traitement comporte en outre un mécanisme d'entraînement configuré pour entraîner en rotation le support rotatif autour du premier axe de rotation.

Selon un mode de réalisation, le mécanisme d'entraînement comporte un actionneur électrique, tel qu'un moteur électrique, un actionneur pneumatique, ou un mécanisme d'entraînement manuel par exemple pourvu d'un levier d'actionnement.

Selon un mode de réalisation, le système de traitement comporte une pluralité dispositifs de maintien, chaque dispositif de maintien étant associé à un emplacement de réception respectif et étant configuré pour maintenir en position une poche d'aspiration reçue dans l'emplacement de réception respectif lors de la découpe de ladite poche d'aspiration par l'organe de coupe.

Selon un mode de réalisation, chaque dispositif de maintien du système de traitement comporte au moins une ouverture de passage qui est sensiblement verticale et qui est configurée pour permettre le passage de l'organe de coupe.

Selon un mode de réalisation, chaque dispositif de maintien comporte une pluralité de plots de maintien. Par exemple, chaque plot de maintien s'étend depuis une face inférieure du support rotatif, et s'étend sensiblement verticalement en direction du sol.

Selon un mode de réalisation, chaque emplacement de réception comporte une surface de support supérieure qui s'étend sensiblement horizontalement et sur laquelle est destinée à reposer au moins en partie une poche d'aspiration reçue dans ledit emplacement de réception.

Selon un mode de réalisation, le bâti comporte une partie de support qui est située sensiblement dans le même plan que les surfaces de support supérieures et qui est configurée pour supporter au moins en partie les poches d'aspiration lors de leurs déplacements dans la zone de découpe. Ainsi, lorsqu'une poche d'aspiration est déplacée dans la zone de découpe, ladite poche d'aspiration est supportée en partie par la surface de support supérieure de l'emplacement de réception respectif et en partie par la partie de support, ce qui permet de maintenir ladite poche d'aspiration sensiblement verticalement lors de son déplacement dans la zone de découpe.

Selon un mode de réalisation, le système de traitement comprend en outre au moins un dispositif de traitement de déchets liquides, ledit dispositif de traitement de déchets liquides étant disposé dans la zone de traitement et étant configuré pour collecter et traiter des déchets liquides provenant de poches d'aspiration découpées dans la zone de découpe.

Selon un mode de réalisation, le dispositif de traitement de déchets liquides comporte un compartiment configuré pour contenir des déchets liquides provenant des poches d'aspiration découpées dans la zone de découpe.

Selon un mode de réalisation, le dispositif de traitement de déchets liquides comporte une source d'agent biocide et est configuré pour injecter de l'agent biocide provenant de la source d'agent biocide dans le compartiment. De façon avantageuse, le dispositif de traitement de déchets liquides est configuré pour injecter de l'agent biocide provenant de la source d'agent biocide dans le compartiment lorsque le compartiment contient un niveau de liquide prédéterminé.

Selon un mode de réalisation, le dispositif de traitement de déchets liquides comporte un compartiment additionnel configuré pour contenir des liquides provenant des proches d'aspiration découpées dans la zone de découpe.

Selon un mode de réalisation, le dispositif de traitement de déchets liquides est configuré pour diriger au moins une partie des déchets liquides, provenant de poches d'aspiration découpées, vers le compartiment additionnel.

Selon un mode de réalisation, le système de traitement comporte des moyens d'évacuation configurés pour évacuer les déchets liquides contenus dans le compartiment et/ou le compartiment additionnel respectivement hors du compartiment et/ou du compartiment additionnel. De façon avantageuse, les moyens d'évacuation sont disposés dans le bâti.

Selon un mode de réalisation, les moyens d'évacuation sont configurés pour évacuer les déchets liquides contenus dans le compartiment et/ou le compartiment additionnel par écoulement gravitaire, par injection d'air comprimé respectivement dans le compartiment et/ou le compartiment additionnel ou encore par génération d'une dépression au niveau d'une ouverture d'évacuation prévue sur le compartiment ou le compartiment additionnel.

Une telle configuration du dispositif de traitement de déchets liquides permet d'assurer un temps de contact adapté entre les déchets liquides et l'agent biocide dans le compartiment et/ou le compartiment additionnel. Une fois le temps de contact entre les déchets liquides collectés et l'agent biocide atteint, il est possible de procéder à l'évacuation des déchets liquides traités hors du compartiment et/ou du compartiment additionnel.

Selon un mode de réalisation, le système de traitement comprend en outre un dispositif de stockage disposé dans la zone de stockage et configuré pour réceptionner et stocker une ou plusieurs poches d'aspiration traitées, ledit dispositif de stockage étant au moins partiellement ouvert vers le haut.

Selon un mode de réalisation, le bâti et le support rotatif délimitent une ouverture d'évacuation située sensiblement à la verticale de la zone de stockage, l'ouverture d'évacuation étant configurée pour autoriser la chute par gravité d'une poche d'aspiration, préalablement traitée, dans le dispositif de stockage lorsque l'emplacement de réception respectif est situé sensiblement à la verticale de la zone de stockage.

Selon un autre mode de réalisation, le système de traitement comporte un ou plusieurs moyens d'éjection configurés pour éjecter une poche d'aspiration traitée hors du support rotatif lorsque l'emplacement de réception respectif est situé en regard de la zone de stockage.

Selon un mode de réalisation, le système de traitement comprend en outre un couvercle monté mobile sur une partie supérieure du bâti entre une position d'ouverture dans laquelle le couvercle autorise un accès au support rotatif et une position de fermeture dans laquelle le couvercle empêche un accès au support rotatif. De façon avantageuse, le couvercle est configuré pour recouvrir au moins en partie l'ouverture d'insertion supérieure lorsque le couvercle occupe la position de fermeture, et pour libérer au moins en partie l'ouverture d'insertion supérieure lorsque le couvercle occupe la position d'ouverture.

Selon un mode de réalisation, le couvercle est monté pivotant autour d'un axe de pivotement qui est sensiblement horizontal.

Selon un mode de réalisation de l'invention, le système de traitement, chacun des emplacements de réception comporte un dispositif de préhension configuré pour occuper alternativement une position de maintien dans lequel le dispositif de préhension maintient en position la poche d'aspiration positionnée dans l'un des emplacements de réception, et une position de libération configurée pour libérer la poche d'aspiration.

Selon un mode de réalisation de l'invention, le dispositif de préhension est configuré pour occuper alternativement une position de maintien dans lequel la poche d'aspiration repose sur une face supérieure du dispositif de préhension afin de maintenir en position la poche d'aspiration à l'intérieur de l'emplacement de réception respectif, et une position de libération dans lequel la poche d'aspiration ne peux pas reposer sur la face supérieure du dispositif de préhension.

Selon un mode de réalisation de l'invention, chacun des dispositifs de préhension comportent en outre une première partie de pince et une deuxième partie de pince.

Selon un mode de réalisation de l'invention, le système de traitement comporte en outre un dispositif d'actionnement configuré pour activer la position de libération de chacun des dispositifs de préhension lorsque l'emplacement de réception respectif est positionné dans une position prédéterminée.

Selon un mode de réalisation de l'invention, le dispositif d'actionnement peut être un chemin de came par exemple.

Selon un mode de réalisation de l'invention, la position prédéterminée est située dans la zone de stockage.

### Brève description des figures

La présente invention sera bien comprise à l'aide de la description qui suit en référence aux figures annexée, dans lesquelles des signes de références identiques correspondent à des éléments structurellement et/ou fonctionnellement identiques ou similaires.
[Fig 1] est une vue éclatée en perspective d'un système de traitement selon la présente invention ;
[Fig 2] est une vue partielle en perspective de dessus du système de traitement de la figure 1 ;
[Fig 3] est une vue en perspective de dessus d'un support rotatif et d'une zone de découpe du système de traitement de la figure 1 ;
[Fig 4] est une vue en perspective de dessus du support rotatif de la figure 3 ;
[Fig 5] est une vue en perspective de côté du support rotatif de la figure 3 et d'un organe de coupe du système de traitement de la figure 1 ;
[Fig 6] est une autre vue en perspective de côté du support rotatif de la figure 3 et de l'organe de coupe de la figure 5 ;
[Fig 7] est une vue en perspective de dessus de la zone de découpe du système de traitement de la figure 1 ;
[Fig 8] est une vue en perspective de dessus de la zone de découpe et d'un dispositif de traitement de déchets liquides du système de traitement de la figure 1 ;
[Fig 9] est une vue de dessous du support rotatif de la figure 3 et de l'organe de coupe de la figure 5 ;
[Fig 10] est une autre vue éclatée en perspective du système de traitement de la figure 1 ;
[Fig 11] est une vue en perspective du système de traitement selon un second mode de réalisation de l'invention ;
[Fig 12] est une vue en perspective de dessous du support rotatif du système de traitement de la figure 11 ;
[Fig 13] est une vue en perspective de dessus du dispositif d'actionnement utilisé dans le mode de réalisation de la figure 11.

### Description détaillée

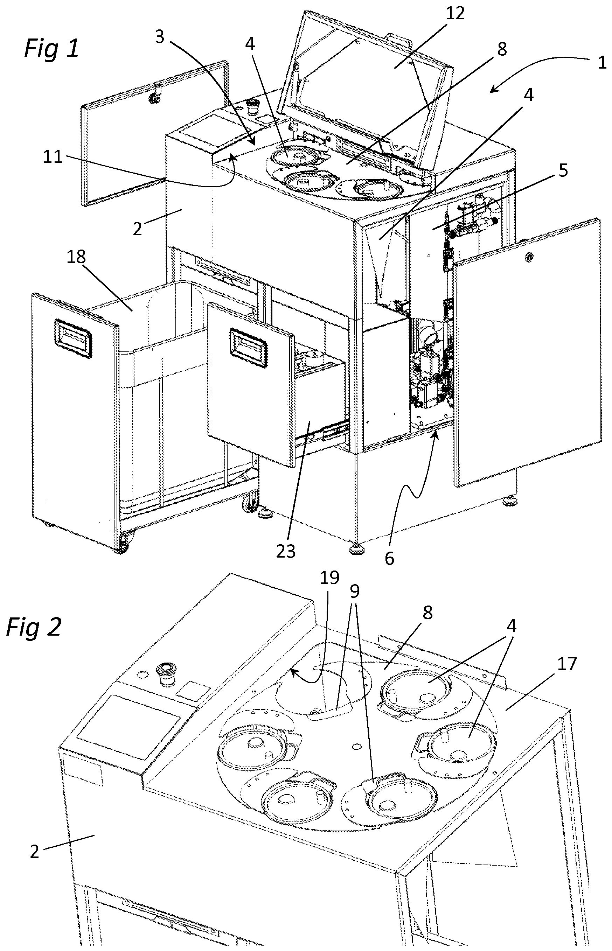

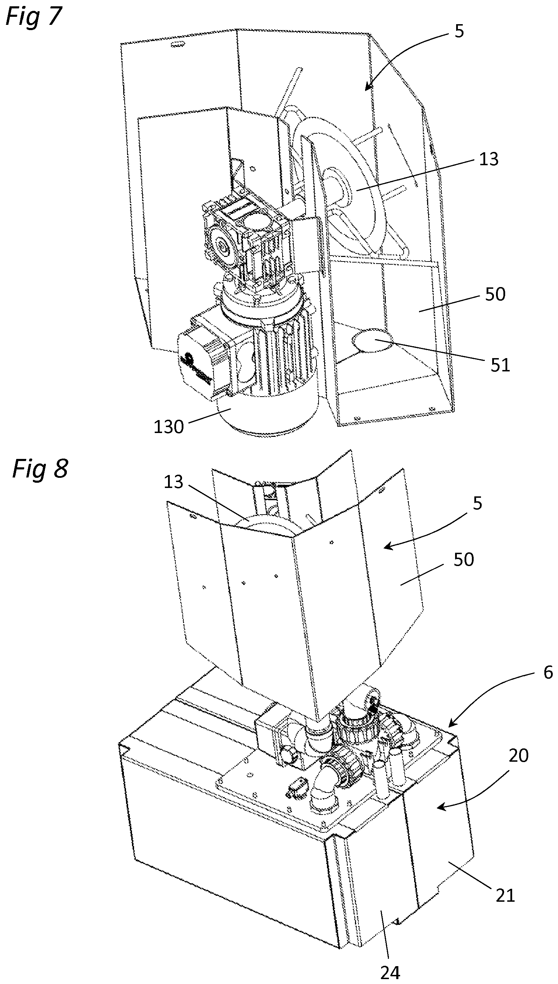

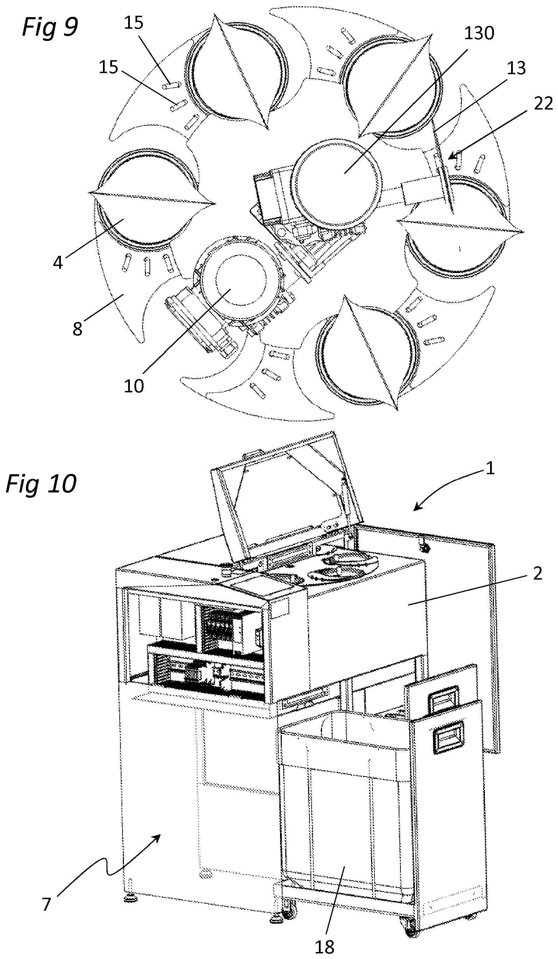

Les figures 1 à 10 représentent un système de traitement 1 configuré pour traiter des poches d'aspiration 4, et plus particulièrement des poches d'aspiration 4 contenant des fluides corporels à traiter, tels que du sang, de l'urine.

Le système de traitement 1 comprend un bâti 2 qui peut par exemple présenter une forme globalement parallélépipédique. Le bâti 2 comporte notamment une zone de chargement 3 configurée pour permettre un chargement de poches d'aspiration 4 au sein du bâti 2. Le bâti 2 comporte également une zone de découpe 5 dans laquelle sont destinées à être découpées des poches d'aspiration 4, et une zone de traitement 6 dans laquelle des déchets liquides, provenant de poches d'aspiration 4 découpées, sont destinés à être traités. Le bâti 2 comporte en outre une zone de stockage 7 dans laquelle sont destinées à être stockées des poches d'aspiration 4 traitées.

Comme montré plus particulièrement sur les figures 2 à 6, le système de traitement 1 comporte en outre un support rotatif 8 qui est logé au moins en partie dans le bâti 2 et qui est monté mobile en rotation autour d'un premier axe de rotation A1. Selon le mode de réalisation visible sur les figures, le premier axe de rotation A1 est sensiblement vertical.

Le support rotatif 8 comprend plusieurs emplacements de réception 9 configurés chacun pour accueillir une poche d'aspiration 4. Le support rotatif 8 est configuré pour déplacer des poches d'aspiration 4 reçues dans les emplacements de réception 9 depuis la zone de chargement 3 vers la zone de découpe 5 puis vers la zone de stockage 7.

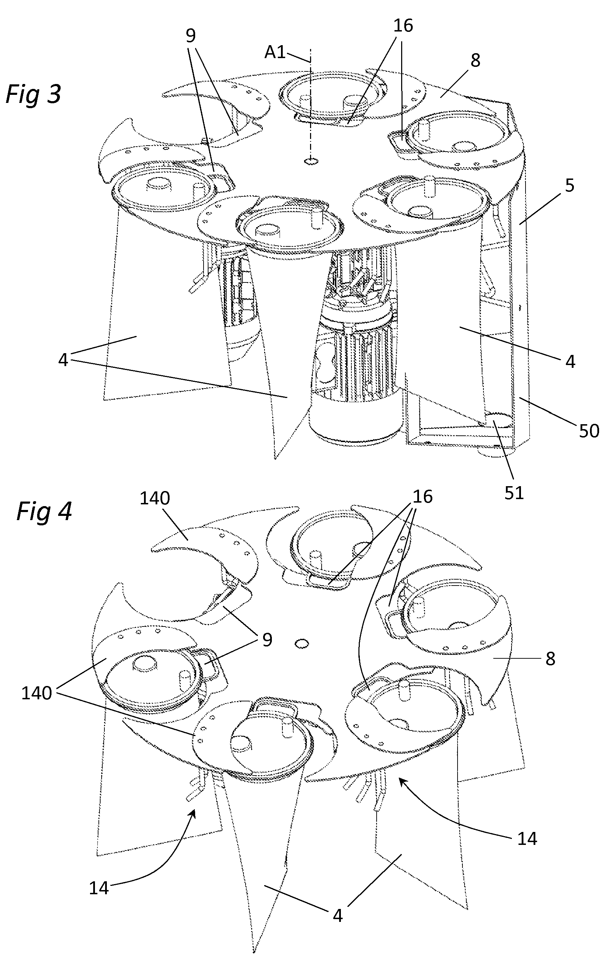

Comme montré plus particulièrement sur la figure 3, les emplacements de réception 9 sont répartis, et avantageusement régulièrement répartis, autour du premier axe de rotation A1, et sont situés à une même distance du premier axe de rotation A1. Le système de traitement 1 peut comporter entre deux et dix emplacements de réception 9, et par exemple six emplacements de réception 9.

Le système de rotation 1 comporte en outre un mécanisme d'entraînement configuré pour entraîner en rotation le support rotatif 8 autour du premier axe de rotation A1. Le mécanisme d'entraînement peut par exemple comporter un actionneur électrique, tel qu'un moteur électrique 10, un actionneur pneumatique, ou encore un mécanisme d'entraînement manuel, tel qu'un levier d'actionnement.

Selon le mode de réalisation représenté sur les figures, la zone de chargement 3 comporte une ouverture d'insertion supérieure 11 configurée pour permettre de positionner simultanément une pluralité de poches d'aspiration 4 dans les emplacements de réception 9 du support rotatif 8 qui sont situés en regard de l'ouverture d'insertion supérieure 11. Cependant, la zone de chargement 3 pourrait comporter plusieurs ouvertures d'insertion supérieures 11 présentant chacune des dimensions correspondant sensiblement à celles des emplacements de réception 9, et permettant ainsi de positionner simultanément une pluralité de poches d'aspiration 4, par exemple trois, dans des emplacements de réception 9 du support rotatif 8 qui sont respectivement situés en regard des ouvertures d'insertion supérieures 11.

Selon le mode de réalisation représenté sur les figures, le système de traitement 1 comprend en outre un couvercle 12 monté mobile sur une partie supérieure du bâti 2 entre une position de fermeture dans laquelle le couvercle 12 recouvre au moins en partie l'ouverture d'insertion supérieure 11 et empêche l'accès au support rotatif 8 et donc aux emplacements de réception 9 situés en regard de l'ouverture d'insertion supérieure 11, et une position d'ouverture dans laquelle le couvercle 12 libère au moins en partie l'ouverture d'insertion supérieure 11 et autorise un accès au support rotatif 8 et donc aux emplacements de réception 9 situés en regard de l'ouverture d'insertion supérieure 11. De façon avantageuse, le couvercle 12 est monté pivotant autour d'un axe de pivotement qui est horizontal qui est situé à proximité d'une face supérieure du bâti 2.

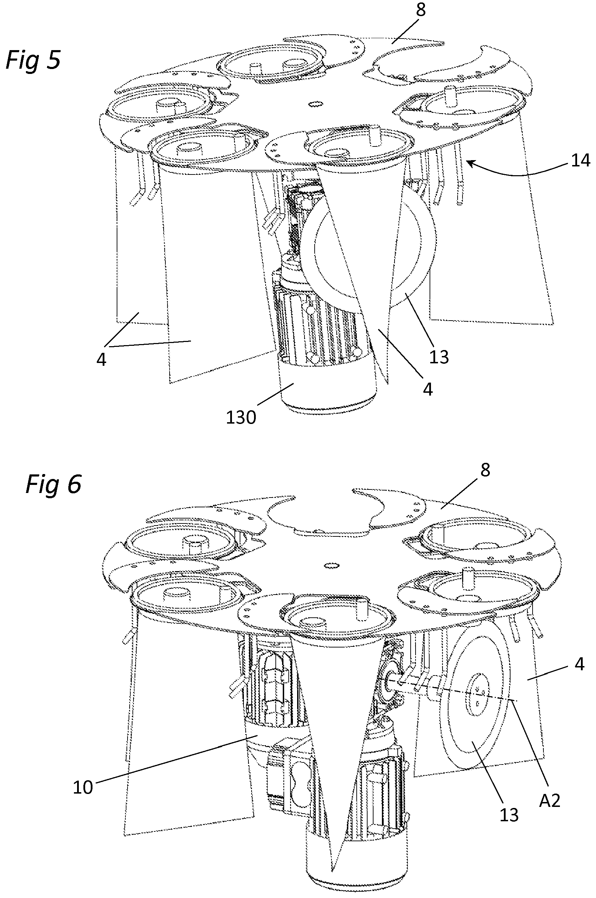

Comme montré plus particulièrement sur les figures 5 à 7, le système de traitement 1 comporte également un organe de coupe 13 qui est situé dans la zone de découpe 5 et qui est monté mobile en rotation autour d'un deuxième axe de rotation A2 qui est sensiblement horizontal. L'organe de coupe 13 définit un plan de coupe qui est sensiblement vertical et qui est configuré pour découper une poche d'aspiration 4 reçue dans un emplacement de réception 9 et située dans la zone de découpe 5. De façon avantageuse, le plan de coupe définit par l'organe de coupe 13 est tangent avec un cercle virtuel dont le centre est situé sur le premier axe de rotation A1 du support rotatif 8. Le système de traitement 1 comporte avantageusement un moteur d'entraînement électrique 130 configuré pour entraîner en rotation l'organe de coupe 13.

Le système de traitement 1 comporte en outre un collecteur 50 définissant la zone de découpe 5, et configuré pour collecter les déchets liquides tombant par gravité des poches découpées par l'organe de coupe 13.

Selon le mode de réalisation représenté sur les figures, le système de traitement 1 comporte également une pluralité de dispositifs de maintien 14. Chaque dispositif de maintien 14 est associé à un emplacement de réception 9 respectif, et est configuré pour maintenir en position une poche d'aspiration 4 reçue dans l'emplacement de réception 9 respectif lors de la découpe de ladite poche d'aspiration 4 par l'organe de coupe 13. De façon avantageuse, chaque dispositif de maintien 14 comporte une ouverture de passage 22 qui est sensiblement verticale et qui est configurée pour permettre le passage de l'organe de coupe 13.

Selon le mode de réalisation représenté sur les figures, chaque dispositif de maintien 14 comporte une pluralité de plots de maintien 15, et par exemple trois plots de maintien 15, configuré pour coopérer avec une paroi latérale d'une poche d'aspiration reçue dans l'emplacement de réception 9 respectif. Chaque plot de maintien 15 s'étend depuis une face inférieure du support rotatif 8, et s'étend sensiblement verticalement en direction du sol.

Selon le mode de réalisation représenté sur les figures, chaque dispositif de maintien 14 comporte en outre un organe d'appui 140 configuré pour recouvrir partiellement une poche d'aspiration 4 reçue dans l'emplacement de réception 9 respectif, et plus particulièrement pour coopérer avec une face supérieure de ladite poche d'aspiration 4 de manière à forme une butée verticale. De façon avantageuse, le support rotatif 8 et le bâti 2 sont configurés pour autoriser, après la mise en place de poches d'aspiration 4 dans leurs emplacements de réception 9 respectifs et la fermeture du couvercle 12, un déplacement relatif entre le support rotatif 8 et lesdites poches d'aspiration 4 lors de la première phase de rotation du support rotatif 8, et ce de telle sorte que chacune des poches d'aspiration 4 venant d'être positionnées sur le support rotatif 8 soit déplacée, par rapport au support rotatif 8, d'une première position (voir la figure 3) dans laquelle l'organe d'appui 140 respectif est décalé par rapport à la poche d'aspiration respective à une deuxième position (voir la figure 4) dans laquelle l'organe d'appui 140 respectif recouvre partiellement la poche d'aspiration respective.

Comme montré plus particulièrement sur la figure 4, chaque emplacement de réception 9 comporte une surface de support supérieure 16 qui s'étend sensiblement horizontalement et sur laquelle est destinée à reposer en partie une poche d'aspiration 4 reçue dans ledit emplacement de réception 9.

Comme montré sur la figure 2, le bâti 2 comporte une partie de support 17 qui est située sensiblement dans le même plan que les surfaces de support supérieures 16 et qui est configurée pour supporter en partie les poches d'aspiration 4 notamment lors de leurs déplacements dans la zone de découpe 5. Ainsi, lorsqu'une poche d'aspiration 4 est déplacée dans la zone de découpe 5, ladite poche d'aspiration 4 est supportée en partie par la surface de support supérieure 16 de l'emplacement de réception 9 respectif et en partie par la partie de support 17, ce qui permet de maintenir ladite poche d'aspiration 4 sensiblement verticalement lors de son déplacement dans la zone de découpe 5.

Selon le mode de réalisation représenté sur les figures, le système de traitement 1 comporte de plus un dispositif de stockage 18 qui est disposé dans la zone de stockage 7 et qui est configuré pour réceptionner et stocker une ou plusieurs poches d'aspiration 4 traitées. De façon avantageuse, le dispositif de stockage 18 est au moins partiellement ouvert vers le haut.

Selon le mode de réalisation représenté sur les figures, le bâti 2 et le support rotatif 8 délimitent une ouverture d'évacuation 19 qui est située sensiblement à la verticale de la zone de stockage 7, et plus particulièrement à la verticale du dispositif de stockage 18, et qui est configurée pour autoriser la chute par gravité d'une poche d'aspiration 4, préalablement traitée, dans le dispositif de stockage 18 lorsque l'emplacement de réception 9 respectif est situé sensiblement à la verticale de la zone de stockage 7.

Selon un autre mode de réalisation non représenté sur les figures, le système de traitement 1 pourrait comporter un ou plusieurs moyens d'éjection configurés pour éjecter une poche d'aspiration 4 traitée hors du support rotatif 8 lorsque l'emplacement de réception 9 respectif est situé en regard de la zone de stockage 7.

Selon le mode de réalisation représenté sur les figures, le système de traitement 1 comprend en outre au moins un dispositif de traitement de déchets liquides 20 qui est disposé dans la zone de traitement 6 et qui est configuré pour collecter et traiter des déchets liquides provenant de poches d'aspiration 4 découpées dans la zone de découpe 5.

Selon le mode de réalisation représenté sur les figures, le dispositif de traitement de déchets liquides 20 comporte un compartiment 21 configuré pour contenir des déchets liquides provenant des poches d'aspiration 4 découpées dans la zone de découpe 5. Le compartiment 21 est configuré pour être relié fluidiquement au collecteur 50, et plus particulièrement à une ouverture inférieure 51 dudit collecteur 50.

De façon avantageuse, le dispositif de traitement de déchets liquides 20 comporte une source d'agent biocide 23 et est configuré pour injecter de l'agent biocide provenant de la source d'agent biocide 23 dans le compartiment 21. Le dispositif de traitement de déchets liquides 20 est plus particulièrement configuré pour injecter de l'agent biocide provenant de la source d'agent biocide 23 dans le compartiment 21 lorsque le compartiment 21 contient un niveau de liquide prédéterminé.

Selon le mode de réalisation non représenté sur les figures, le dispositif de traitement de déchets liquides 20 comporte un compartiment additionnel 24 configuré également pour contenir des liquides provenant des proches d'aspiration 4 découpées dans la zone de découpe 5. De façon avantageuse, le dispositif de traitement de déchets liquides 20 est configuré pour diriger au moins une partie des déchets liquides, provenant de poches d'aspiration 4 découpées, vers le compartiment additionnel 24 lorsque le compartiment 21 contient un niveau de liquide prédéterminé. A cet effet, le dispositif de traitement de déchets liquides 20 peut par exemple comporter une vanne à trois voies et un actionneur configuré pour déplacer la vanne à trois voies entre deux positions de fonctionnement différentes.

Le système de traitement 1 comporte en outre des moyens d'évacuation (non représentés sur les figures) configurés pour évacuer les déchets liquides traités contenus dans le compartiment 21 et/ou le compartiment additionnel 24. De façon avantageuse, les moyens d'évacuation sont disposés dans le bâti 2. Les moyens d'évacuation peuvent par exemple être configurés pour évacuer les déchets liquides traités contenus dans le compartiment 21 et/ou le compartiment additionnel 24 par écoulement gravitaire ou par injection d'air comprimé respectivement dans le compartiment 21 et/ou le compartiment additionnel 24.

Une telle configuration du dispositif de traitement de déchets liquides 20 permet d'assurer un temps de contact adapté entre les déchets liquides et l'agent biocide dans le compartiment 21 et/ou le compartiment additionnel 24. Une fois le temps de contact entre les déchets liquides collectés et l'agent biocide atteint, il est possible de procéder à l'évacuation des déchets liquides traités hors du compartiment 21 et/ou du compartiment additionnel 24.

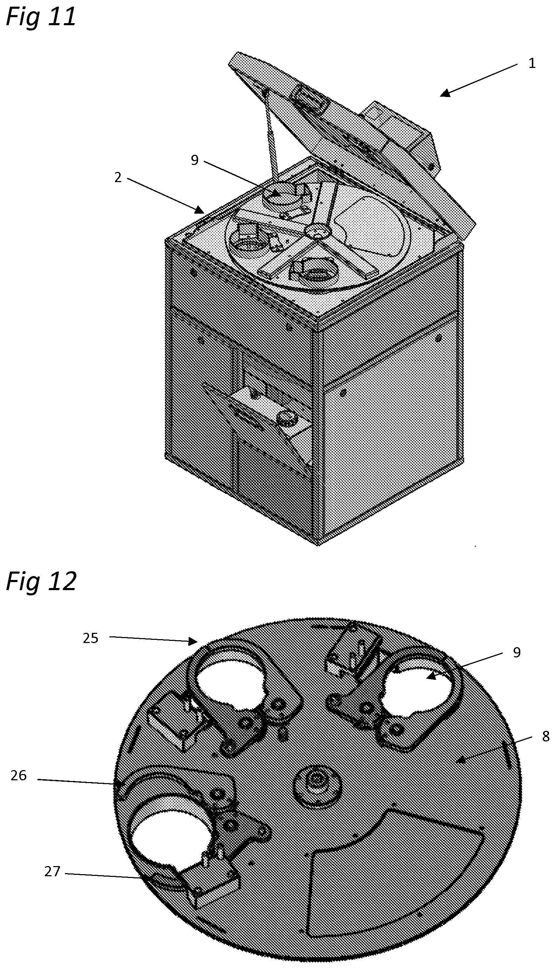

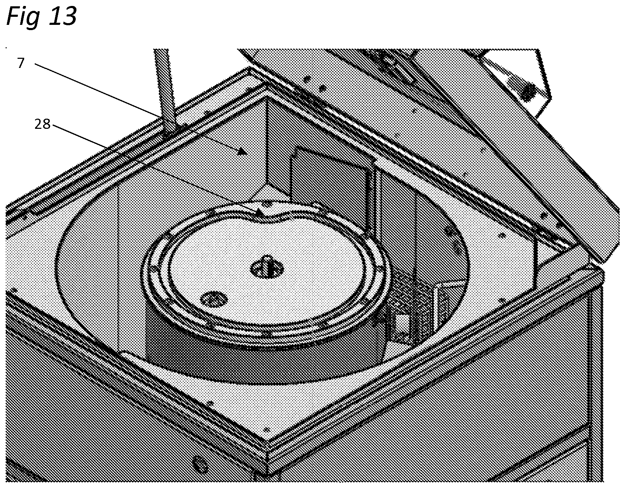

Un second mode de réalisation de l'invention est présenté sur les figures 11 à 13. Le second mode de réalisation de l'invention diffère du premier mode de réalisation en ce que les emplacements de réception 9 du support rotatif 8 comportent chacun un dispositif de préhension 25 configuré pour occuper alternativement une position de maintien et une position de libération. Chacun des dispositifs de préhension 25 comporte une première partie de pince 26 et une deuxième partie de pince 27. Lorsque le dispositif de préhension 25 est dans la position de maintien, la poche d'aspiration 4, positionnée dans l'emplacement de réception 9 respectif, repose sur une partie supérieure du dispositif de préhension 25 formée par la première partie de pince 26 et de la deuxième partie de pince 27. De façon inverse, lorsque le dispositif de préhension 25 est dans la position de libération, la deuxième partie de pince 27 n'est plus en contact avec la première partie de pince 26 ce qui empêche la poche d'aspiration 4 de reposer sur la partie supérieure du dispositif de préhension 25.

Dans ce second mode de réalisation de l'invention, le système de traitement 1 comporte en outre un dispositif d'actionnement 28, tel qu'un chemin de came par exemple, lequel est configuré pour activer et désactiver la position de libération de chacun des dispositifs de préhension 25 lorsque l'emplacement de réception 9 respectif est positionné dans une position prédéterminée. Avantageusement, la position prédéterminée est prévue dans la zone de stockage 7 et à l'aplomb du dispositif de stockage 18 de sorte que chacune des poches d'aspiration 4 soit libérée du dispositif de préhension 25 afin de tomber par gravité à l'intérieur du dispositif de stockage 18.

Bien entendu, la présente invention n'est nullement limitée au mode de réalisation décrit et limité qui n'a été donné qu'à titre d'exemple. Des modifications restent possibles, notamment du point de vue de la constitution des divers éléments, pour autant qu'elles relèvent de la portée des revendications.

## Revendications

1. Système de traitement (1) de poches d'aspiration (4), comprenant :
- un bâti (2) comportant une zone de chargement (3) configurée pour permettre un chargement de poches d'aspiration (4) au sein du bâti (2), une zone de découpe (5) dans laquelle sont destinées à être découpées des poches d'aspiration (4), une zone de traitement (6) dans laquelle des déchets liquides, provenant de poches d'aspiration (4) découpées, sont destinés à être traités, et une zone de stockage (7) dans laquelle sont destinées à être stockées des poches d'aspiration (4) traitées,
- un support rotatif (8) qui est logé au moins en partie dans le bâti (2) et qui est monté mobile en rotation autour d'un premier axe de rotation (A1), le support rotatif (8) comprenant plusieurs emplacements de réception (9) configurés chacun pour accueillir une poche d'aspiration (4), le support rotatif (8) étant configuré pour déplacer des poches d'aspiration (4) reçues dans les emplacements de réception (9) depuis la zone de chargement (3) vers la zone de découpe (5) puis vers la zone de stockage (7),
- un organe de coupe (13) qui est situé dans la zone de découpe (5) et qui est monté mobile en rotation autour d'un deuxième axe de rotation (A2) qui est sensiblement horizontal, l'organe de coupe (13) définissant un plan de coupe qui est sensiblement vertical et étant configuré pour découper au moins une poche d'aspiration (4) reçue dans un emplacement de réception (9) et située dans la zone de découpe (5).

2. Système de traitement (1) selon la revendication1, dans lequel le premier axe de rotation (A1) est sensiblement vertical.

3. Système de traitement (1) selon l'une quelconque des revendications précédentes, dans lequel la zone de chargement (3) comporte au moins une ouverture d'insertion supérieure (11) configurée pour permettre de positionner simultanément une pluralité de poches d'aspiration (4) dans des emplacements de réception (9) du support rotatif (8) situés en regard de l'au moins une ouverture d'insertion supérieure (11).

4. Système de traitement (1) selon l'une quelconque des revendications précédentes, dans lequel les emplacements de réception (9) sont angulairement décalés les uns par rapport aux autres vis-à-vis du premier axe de rotation (A1).

5. Système de traitement (1) selon l'une quelconque des revendications précédentes, lequel comporte en outre un mécanisme d'entraînement configuré pour entraîner en rotation le support rotatif (8) autour du premier axe de rotation (A1).

6. Système de traitement (1) selon l'une quelconque des revendications précédentes, dans lequel le support rotatif (8) comporte une pluralité d'organes de maintien (14), chaque organe de maintien (14) étant associé à un emplacement de réception (9) respectif et étant configuré pour maintenir en position une poche d'aspiration (4) reçue dans l'emplacement de réception (9) respectif lors de la découpe de ladite poche d'aspiration (4) par l'organe de coupe (13).

7. Système de traitement (1) selon la revendication précédente, chaque organe de maintien (14) comporte au moins une ouverture de passage qui est sensiblement verticale et qui est configurée pour permettre le passage de l'organe de coupe (13).

8. Système de traitement (1) selon l'une quelconque des revendications précédentes, lequel comprend en outre au moins un dispositif de traitement de déchets liquides (20), ledit dispositif de traitement de déchets liquides (20) étant disposé dans la zone de traitement (6) et configuré pour collecter et traiter des déchets liquides provenant de poches d'aspiration (4) découpées dans la zone de découpe (5).

9. Système de traitement (1) selon l'une quelconque des revendications précédentes, lequel comprend en outre un dispositif de stockage (18) disposé dans la zone de stockage (7) et configuré pour réceptionner et stocker une ou plusieurs poches d'aspiration (4) traitées, ledit dispositif de stockage (18) étant au moins partiellement ouvert vers le haut.

10. Système de traitement (1) selon la revendication précédente, dans lequel le bâti (2) et le support rotatif (8) délimitent une ouverture d'évacuation (19) située sensiblement à la verticale de la zone de stockage (7), l'ouverture d'évacuation (19) étant configurée pour autoriser la chute par gravité d'une poche d'aspiration (4), préalablement traitée, dans le dispositif de stockage (18) lorsque l'emplacement de réception (9) respectif est situé sensiblement à la verticale de la zone de stockage (7).

11. Système de traitement (1) selon l'une quelconque des revendications précédentes, lequel comprend en outre un couvercle (12) monté mobile sur une partie supérieure du bâti (2) entre une position d'ouverture dans laquelle le couvercle (12) autorise un accès au support rotatif (8) et une position de fermeture dans laquelle le couvercle (12) empêche un accès au support rotatif (8).

12. Système de traitement (1) selon l'une quelconque des revendications précédentes, dans lequel chacun des emplacements de réception (9) comportent un dispositif de préhension (25) configuré pour occuper alternativement une position de maintien dans lequel le dispositif de préhension (25) maintient en position la poche d'aspiration (4) positionnée dans l'un des emplacements de réception (9), et une position de libération configurée pour libérer la poche d'aspiration (4).

## Patentansprüche

1. Behandlungssystem (1) für Saugbeutel (4), das Folgendes umfasst:
- ein Gestell (2), das einen Ladebereich (3), der so eingerichtet ist, dass er ein Laden von Saugbeuteln (4) innerhalb des Gestells (2) ermöglicht, einen Schneidebereich (5), in dem Saugbeutel (4) geschnitten werden sollen, einen Behandlungsbereich (6), in dem flüssige Abfälle, die aus geschnittenen Saugbeuteln (4) stammen, beehandelt werden sollen, und einen Lagerbereich (7) enthält, in dem die behandelten Saugbeutel (4) gelagert werden sollen,
- einen drehbaren Träger (8), der mindestens teilweise im Gestell (2) untergebracht ist und der drehbeweglich um eine erste Drehachse (A1) montiert ist, wobei der drehbare Träger (8) mehrere Aufnahmeplätze (9) umfasst, die jeweils so eingerichtet sind, dass sie einen Saugbeutel (4) aufnehmen, wobei der drehbare Träger (8) so eingerichtet ist, dass er die in den Aufnahmeplätzen (9) aufgenommenen Saugbeutel (4) vom Ladebereich (3) zum Schneidebereich (5) und dann zum Lagerbereich (7) bewegt,
- ein Schneideelement (13), das sich im Schneidebereich (5) befindet und das drehbeweglich um eine zweite Drehachse (A2) montiert ist, die im Wesentlichen horizontal ist, wobei das Schneideelement (13) eine Schnittebene definiert, die im Wesentlichen vertikal ist und so eingerichtet ist, dass sie mindestens einen Saugbeutel (4) schneidet, der in einem Aufnahmeplatz (9) aufgenommen ist und sich im Schneidebereich (5) befindet.

2. Behandlungssystem (1) nach Anspruch 1, wobei die erste Drehachse (A1) im Wesentlichen vertikal ist.

3. Behandlungssystem (1) nach einem der vorhergehenden Ansprüche, wobei der Ladebereich (3) mindestens eine obere Einführöffnung (11) enthält, die so eingerichtet ist, dass sie es ermöglicht, eine Vielzahl von Saugbeuteln (4) gleichzeitig in den Aufnahmeplätzen (9) des drehbaren Trägers (8) zu positionieren, die sich gegenüber der mindestens einen oberen Einführöffnung (11) befinden.

4. Behandlungssystem (1) nach einem der vorhergehenden Ansprüche, wobei die Aufnahmeplätze (9) gegenüber der ersten Drehachse (A1) winkelförmig zueinander versetzt sind.

5. Behandlungssystem (1) nach einem der vorhergehenden Ansprüche, das ferner einen Antriebsmechanismus enthält, der so eingerichtet ist, dass er den drehbaren Träger (8) drehbar um die erste Drehachse (A1) antreibt.

6. Behandlungssystem (1) nach einem der vorhergehenden Ansprüche, wobei der drehbare Träger (8) eine Vielzahl von Halteelementen (14) enthält, wobei jedes Halteelement (14) mit einem jeweiligen Aufnahmeplatz (9) verbunden und so eingerichtet ist, dass es einen in dem jeweiligen Aufnahmeplatz (9) aufgenommenen Saugbeutel (4) beim Schneiden des Saugbeutels (4) durch das Schneidelement (13) in Position hält.

7. Behandlungssystem (1) nach dem vorhergehenden Anspruch, wobei jedes Halteelement (14) mindestens eine Durchgangsöffnung enthält, die im Wesentlichen vertikal ist und so eingerichtet ist, dass sie den Durchgang des Schneidelements (13) ermöglicht.

8. Behandlungssystem (1) nach einem der vorhergehenden Ansprüche, das ferner mindestens eine Vorrichtung zur Behandlung von flüssigen Abfällen (20) umfasst, wobei die Vorrichtung zur Behandlung von flüssigen Abfällen (20) in dem Behandlungsbereich (6) angeordnet und so eingerichtet ist, dass sie flüssige Abfälle aus Saugbeuteln (4), die in dem Schneidebereich (5) geschnitten wurden, aufnimmt und behandelt.

9. Behandlungssystem (1) nach einem der vorhergehenden Ansprüche, das ferner eine Lagervorrichtung (18) umfasst, die im Lagerbereich (7) angeordnet und so eingerichtet ist, dass sie einen oder mehrere behandelte Saugbeutel (4) aufnimmt und lagert, wobei die Lagervorrichtung (18) mindestens teilweise nach oben geöffnet ist.

10. Behandlungssystem (1) nach dem vorhergehenden Anspruch, wobei das Gestell (2) und die drehbare Träger (8) eine Abflussöffnung (19) begrenzen, die sich im Wesentlichen vertikal zum Lagerbereich (7) befindet, wobei die Abflussöffnung (19) so eingerichtet ist, dass sie das Fallen eines zuvor behandelten Saugbeutels (4) durch die Schwerkraft in die Lagervorrichtung (18) zulässt, wenn sich der jeweilige Aufnahmeplatz (9) im Wesentlichen vertikal zum Lagerbereich (7) befindet.

11. Behandlungssystem (1) nach einem der vorhergehenden Ansprüche, das ferner einen Deckel (12) umfasst, der an einem oberen Teil des Gestells (2) beweglich zwischen einer Öffnungsposition, in der der Deckel (12) einen Zugang zum drehbaren Träger (8) zulässt, und einer Schließposition, in der der Deckel (12) einen Zugang zum drehbaren Tträger (8) verhindert, montiert ist.

12. Behandlungssystem (1) nach einem der vorhergehenden Ansprüche, wobei jeder der Aufnahmeplätze (9) eine Greifvorrichtung (25) enthält, die so eingerichtet ist, dass sie abwechselnd eine Halteposition, in der die Greifvorrichtung (25) den in einem der Aufnahmeplätze (9) positionierten Saugbeutel (4) in Position hält, und eine Freigabeposition einnimmt, die so eingerichtet ist, dass sie den Saugbeutel (4) freigibt.

## Claims

1. A treatment system (1) for suction bags (4), comprising:
- a frame (2) including a loading area (3) configured to allow loading of suction bags (4) within the frame (2), a cutting area (5) in which suction bags (4) are intended to be cut, a treatment area (6) in which liquid waste, originating from cut suction bags (4), is intended to be treated, and a storage area (7) in which treated suction bags (4) are intended to be stored,
- a rotating support (8) which is housed at least partly in the frame (2) and which is mounted movably in rotation about a first axis of rotation (A1), the rotating support (8) comprising several receiving locations (9) each configured to accommodate a suction bag (4), the rotating support (8) being configured to move suction bags (4) received in the receiving locations (9) from the loading area (3) to the cutting area (5) then to the storage area (7),
- a cutting member (13) which is located in the cutting area (5) and which is mounted movably in rotation about a second axis of rotation (A2) which is substantially horizontal, the cutting member (13) defining a cutting plane which is substantially vertical and being configured to cut at least one suction bag (4) received in a receiving location (9) and located in the cutting area (5).

2. The treatment system (1) according to claim 1, wherein the first axis of rotation (A1) is substantially vertical.

3. The treatment system (1) according to any one of the preceding claims, wherein the loading area (3) includes at least one upper insertion opening (11) configured to allow a plurality of suction bags (4) to be positioned simultaneously in receiving locations (9) of the rotating support (8) located opposite the at least one upper insertion opening (11).

4. The treatment system (1) according to any one of the preceding claims, wherein the receiving locations (9) are angularly offset from each other with respect to the first axis of rotation (A1).

5. The treatment system (1) according to any one of the preceding claims, which further includes a drive mechanism configured to drive in rotation the rotating support (8) about the first axis of rotation (A1).

6. The treatment system (1) according to any one of the preceding claims, wherein the rotating support (8) includes a plurality of holding members (14), each holding member (14) being associated with a respective receiving location (9) and being configured to hold in position a suction bag (4) received in the respective receiving location (9) during the cutting of said suction bag (4) by the cutting member (13).

7. The treatment system (1) according to the preceding claim, each holding member (14) includes at least one passage opening which is substantially vertical and which is configured to allow the passage of the cutting member (13).

8. The treatment system (1) according to any one of the preceding claims, which further comprises at least one liquid waste treatment device (20), said liquid waste treatment device (20) being disposed in the treatment area (6) and configured to collect and treat liquid waste originating from suction bags (4) cut in the cutting area (5).

9. The treatment system (1) according to any one of the preceding claims, which further comprises a storage device (18) disposed in the storage area (7) and configured to receive and store one or several treated suction bags (4), said storage device (18) being at least partially open upwards.

10. The treatment system (1) according to the preceding claim, wherein the frame (2) and the rotating support (8) delimit a discharge opening (19) located substantially at the vertical of the storage area (7), the discharge opening (19) being configured to allow the fall by gravity of a suction bag (4), previously treated, into the storage device (18) when the respective receiving location (9) is located substantially at the vertical of the storage area (7).

11. The treatment system (1) according to any one of the preceding claims, which further comprises a cover (12) movably mounted on an upper part of the frame (2) between an open position in which the cover (12) allows access to the rotating support (8) and a closed position in which the cover (12) prevents access to the rotating support (8).

12. The treatment system (1) according to any one of the preceding claims, wherein each of the receiving locations (9) includes a gripping device (25) configured to alternately occupy a holding position in which the gripping device (25) holds in position the suction bag (4) positioned in one of the receiving locations (9), and a release position configured to release the suction bag (4).
